Europäisches Patentamt

European Patent Office

Office européen des brevets

(11) Veröffentlichungsnummer: **0 516 958 A2**

# EUROPÄISCHE PATENTANMELDUNG

(21) Anmeldenummer: **92106761.7**

(22) Anmeldetag: **21.04.92**

(51) Int. Cl.5: **C12N  15/54**, C12N 15/82, C12N 1/21, C12N 5/10, A01N 65/00, C12Q 1/68

(30) Priorität: **30.05.91 DE 4117747**

(43) Veröffentlichungstag der Anmeldung: **09.12.92 Patentblatt  92/50**

(84) Benannte Vertragsstaaten: **BE CH DE DK FR GB IT LI NL SE**

(71) Anmelder: **BAYER AG**

**W-5090 Leverkusen 1 Bayerwerk(DE)**

(72) Erfinder: **Matern, Ulrich, Professor Dr.**
**Gerichtsstrasse 1**
**W-7630 Lahr(DE)**
Erfinder: **Hain, Rüdiger, Dr.**
**Talstrasse 53a**
**W-4018 Langenfeld(DE)**
Erfinder: **Reif, Hans-Jörg, Dr.**
**Gottesweg 165**
**W-5000 Köln 41(DE)**
Erfinder: **Stenzel, Klaus, Dr.**
**Seesener Strasse 17**
**W-4000 Düsseldorf 13(DE)**
Erfinder: **Thomzik, Jürgen, Dr.**
**Sauerbruchstrasse 13a**
**W-4018 Langenfeld(DE)**

(54) **Kaffeoyl-CoA 3-O-Methyltransferase-Gene.**

(57)   Die vorliegende Erfindung betrifft neue aus Pflanzen isolierte Kaffeoyl-CoA 3-O-Methyltransferase-Gene und ihre Verwendung zur Transformation von Vektoren, Wirtsorganismen und Pflanzen sowie zur Erzeugung von Pflanzen, welche eine erhöhte Resistenz gegenüber Schädlingen aufweisen.

EP 0 516 958 A2

Die vorliegende Erfindung betrifft neue aus Pflanzen isolierte Kaffeoyl-CoA 3-O-Methyltransferase-Gene (im folgenden als CCoAMT-Gene bezeichnet) und ihre Verwendung zur Transformation von Vektoren, Wirtsorganismen und Pflanzen sowie zur Erzeugung von Pflanzen, welche eine erhöhte Resistenz gegenüber Schädlingen aufweisen.

Das Enzym Kaffeoyl-CoA 3-O-Methyltransferase, im folgenden bezeichnet als CCoAMT, katalysiert die Methylierung von Kaffeoyl-CoA in einem erst kürzlich beschriebenen Biosyntheseweg, der von trans-4-Cumaroyl-CoA zu trans-Feruloyl-CoA führt (Matern, U., und Kneusel, R.E. 1988, Phytoparasitica 16:153-170; Kneusel, R.E., Matern, U., und Nicolay, K. 1989, Arch. Biochem. Biophys. 269:455 bis 462; Pakusch, A.-E., Kneusel, R.E., und Matern, U., 1989, Arch. Biochem. Biophys. 271:488 bis 494).

Pflanzen verstärken unter Pilzbefall sehr rasch ihre Zellwand durch den Einbau von Zimtsäuren, gefolgt von ihrer Vernetzung zu polymeren Strukturen bzw. dem Aufbau von Lignin. Feruloyl-CoA ist unter diesen Bedingungen der bevorzugte Acyldonor sowohl für die Veresterung von Zellwand-Polysacchariden als auch für die Lignifizierung (Reduktion zu Coniferyl Alkohol). Die Geschwindigkeit und das Ausmaß der Zellwandveränderung bestimmen im wesentlichen den Verlauf der Infektion und das Schicksal der Pflanze, wobei die "hypersensitive Reaktion" die völlige Resistenz der Pflanze kennzeichnet, verbunden mit der besonders starken und schnellen Veränderung der Zellwand und dem Tod der direkt betroffenen Zellen. Diese hypersensitive Reaktion wird ebenfalls beobachtet in der Resistenzreaktion von Pflanzen gegen Virusinfektion. Erst kürzlich wurde entdeckt, daß Feruloyl-CoA in vivo nicht in jedem Fall durch Aktivierung der Ferulasäure entsteht, sondern auch durch Umsetzung von Cumaroyl-CoA. Die daran beteiligte Kaffeoyl-CoA-spezifische Methyltransferase besitzt kaum Homologie zu bisher bekannten Enzymen (Pakusch, A.-E., Matern, U., und Schiltz, E., 1991, Plant Physiol. 95:137 bis 143), ist taxonomisch weit verbreitet in Pflanzen und dort durch z.B. Pilzbefall induzierbar.

Ein großer Teil der Welternte von Kulturpflanzen wird ständig durch Schädlinge vernichtet (1967 betrug der Verlust an potentieller Ernte 35 %; vergl. Chemistry of Pesticides, herausgegeben von K.H. Büchel, John Wiley & Sons, New York, 1983, Seite 6). Es besteht somit ein dringendes Bedürfnis, alle Möglichkeiten zu erforschen und nutzbar zu machen, welche geeignet sind, den Schädlingsbefall bei Kulturpflanzen zu vermindern oder zu verhindern.

Es wurden nun die neuen Kaffeoyl-CoA 3-O-Methyltransferase Gene, im folgenden bezeichnet als CCoAMT-Gene gefunden, welche in die Erbmasse (das Genom) von Pflanzen eingebaut werden können, die keine CCoAMT oder nur unzureichend CCoAMT erzeugen, wodurch eine erhöhte Resistenz dieser Pflanzen gegen Schädlinge hervorgerufen werden kann.

Es ist überraschend, daß eine neue Art von Resistenz-Genen gefunden werden konnte, die als fremde oder zusätzliche DNA in das Genom von Pflanzen eingebaut werden kann, wodurch eine erhöhte Schädlingsresistenz bei den erhaltenen transgenen Pflanzen erzielt wird. Ein besonderer Vorzug der vorliegenden Erfindung besteht darin, daß sie - anders als z.B. im Falle der erhöhten Akkumulation von Phytoalexinen - nicht auf die Erzeugung potentiell toxischer Metabolite abzielt. Toxikologische Vorbehalte bestehen auch deshalb nicht, weil in den transformierten Pflanzen die rasche Synthese von überwiegend unlöslichen, antibiotisch unwirksamen Verbindungen angestrebt wird, die als physikalische Barriere fungieren bzw. die eventuelle Pathogen-induzierte, enzymatische Lyse von Zellwand-Polysacchariden durch Acylierung des "Substrates" verhindern sollen. Im Gegensatz zur Transformation von Pflanzen mit Genen lytischer Enzyme wie z.B. Lysozym oder auch Chitinase, die bestenfalls selektiv wirksam werden können, bietet die erhöhte Bereitschaft von Pflanzen zur Verstärkung der Zellwand Schutz gegen jede Form von Pathogenen, einschließlich der Viren, Die hier vorliegende Erfindung verfolgt deshalb ein neuartiges Prinzip des Pflanzenschutzes mit breiter Anwendung.

Unter CCoAMT-Genen soll jede Nukleinsäure (DNA) verstanden werden, die nach ihrer Transkription in RNA und Translation in Protein die Bildung eines Enzyms bewirkt, welches die Eigenschaften einer CCoAMT besitzt, wobei diese Nukleinsäure aus ihrer natürlichen Umgebung isoliert vorliegt oder in einen Vektor integriert ist oder in einer prokaryontischen oder eukaryontischen DNA als "fremde" DNA oder als "zusätzliche" DNA enthalten ist.

Unter CCoAMT-Genen sollen auch solche CCoAMT-Gene verstanden werden, die an ihrem Anfang und/oder Ende noch DNA-Sequenzen enthalten, die die Funktion der Gene nicht oder nicht wesentlich behindern, Diese auch als "Gen-Einheiten" bezeichneten DNA-Sequenzen entstehen, z.B. durch das Herausschneiden mit Restriktionsenzymen, da keine Schnittstellen für übliche Restriktionsenzyme exakt am Beginn und am Ende des Gens vorliegen. Die CCoAMT-Gene bzw. die Gen-Einheiten können auch an ihren Enden solche DNA Sequenzen tragen, welche für ihre Handhabung jeweils angepaßt sind (z.B. "Linker").

Die CCoAMT-Gene (bzw. die Gen-Einheiten) können in der Form vorliegen, wie sie im Genom von Pflanzen enthalten sind ("genomische" Form, einschließlich nicht CCoAMT kodierender und/oder nicht regulatorisch wirkender Sequenzen (wie Introns) oder in einer Form, welche der cDNA ("copy" DNA) entspricht, die über mRNA mit Hilfe von Reverse-Transkriptase/Polymerase erhältlich ist (und keine Introns mehr enthält). Die CCoAMT-Gene können auch in teilweise oder vollständig synthetisierter Form vorliegen. Unter synthetischen Genen werden auch solche verstanden, welche durch das neue Zusammenfügen von Teilen natürlicher Gene entstehen.

In den erfindungsgemäßen CCoAMT-Genen (bzw. den Gen-Einheiten) können DNA-Abschnitte durch im wesentlichen gleichwirkende andere DNA-Abschnitte oder DNA's ersetzt sein.

Im vorliegenden Zusammenhang soll unter "fremder" DNA, solche DNA (insbesondere Gene bzw. Gen-Einheiten oder deren Teile) verstanden werden, welche in einem bestimmten prokaryontischen oder eukaryontischen Genom nicht natürlich vorkommt, sondern erst durch Eingriffe durch den Menschen in dieses Genom aufgenommen wird. "Zusätzliche" DNA (insbesondere Gene bzw. Gen-Einheiten oder deren Teile) soll solche DNA sein, welche in dem jeweiligen prokaryontischen oder eukaryontischen Genom zwar natürlich vorkommt, jedoch in zusätzlicher Menge durch Eingriffe durch den Menschen in dieses Genom aufgenommen wird. Die "fremde" DNA oder "zusätzliche" DNA kann je nach Bedarf und Art des vorliegenden Falles in einem oder mehreren Exemplaren eingebaut werden.

CCoAMT, welche unter Mitwirkung der erfindungsgemäßen CCoAMT-Gene (bzw. der Gen-Einheiten) in Pflanzen oder Pflanzenzellen gebildet wird, bedeutet jedes Enzym, welches wie CCoAMT wirkt und in Pflanzen deren Resistenz gegenüber Schädlingen erhöht.

Die bevorzugten erfindungsgemäßen CCoAMT-Gene sind dadurch gekennzeichnet, daß sie mit der im Plasmid pL2-4 enthaltenen CCoAMT-cDNA-Sequenz oder ihren Teilen bzw. mit der cDNA-Sequenz gemäß SEQ ID No: 1 oder ihren Teilen hybridisieren und für CCoAMT codieren.

Erfindungsgemäß bevorzugte CCoAMT-Gene sind die CCoAMT-Gene, welche in Petersilie (Petroselinum crispum), Karotten (Daucus carota), Nelke (Dianthus caryophyllus) und Saflor (Carthamus tinctorius), besonders bevorzugt in Petersilie, vorkommen und daraus isoliert werden können.

Ganz besonders bevorzugt wird als erfindungsgemäßes CCoAMT-Gen das CCoAMT-Gen, welches (als Gen-Einheit) in Form der cDNA auf dem Plasmid pL2-4, (welches weiter unten näher beschrieben wird) vorliegt, sowie die im wesentlichen gleichwirkenden DNA-Sequenzen.

Die auf dem Plasmid enthaltene cDNA wurde aus Petersilie isoliert. Sie besteht aus einer 370 Nukleotide langen 5' untranslatierten leader-Sequenz und der kompletten proteincodierenden Region von Position 371 bis Position 1093, gefolgt von 67 Nukleotiden 3' untranslatierter Sequenz. Das gesamte Fragment wurde mit EcoRI Linkern an beiden Seiten versehen und in den Vector pGEM 7 (Promega Corp. Madison, Wi., USA) kloniert. Die restliche Sequenz der 3' untranslatierten Region von Position 1160 bis 1258 liegt nicht auf dem Plasmid pL2-4 vor. Diese poly-Adenylierungssequenz kann synthetisch hergestellt werden oder durch eine andere poly-A-Sequenz ersetzt werden. Die komplette cDNA-Sequenz geht aus dem Sequenzprotokoll SEQ ID No:1 hervor.

Der 5' untranslatierte Bereich, die komplette codierende Region und 67 Nukleotide der 3' untranslatierten Region können in üblicher Weise durch EcoRI auf einem ca. 1170 langen Fragment isoliert werden,

Besonders hervorgehoben seien die chimären Genfusionen aus dem TR-Promotor oder dem 35 S-Promotor und der proteincodierenden Region der CCoAMT-Gene, vorzugsweise des Gens aus Petersilie, insbesondere des Gens, das der cDNA auf dem Plasmid pL2-4 entspricht.

Es wurde gefunden, daß die in Pflanzen vorkommenden CCoAMT-Gene über weite Bereiche eine DNA-Sequenzhomologie aufweisen. Die erfindungsgemäßen CCoAMT-Gene können daher auf Grund der Sequenzhomologie mit Hilfe der auf dem Plasmid pL2-4 enthaltenen cDNA oder ihrer Teile oder den Sequenzinformationen gemäß SEQ ID No:1 in üblicher Weise mit den bekannten Methoden der Molekularbiologie aus Pflanzen in einfacher Weise isoliert werden.

Als Pflanzen, aus denen erfindungsgemäße CCoAMT-Gene isoliert werden können, kommen praktisch alle ein- oder zweikeimblättrige Pflanzen, vorzugsweise zweikeimblättrige Pflanzen in Frage, wobei beispielhaft und bevorzugt Petersilie, Karotte, Saflor und Nelke genannt seien,

Wie bereits erwähnt, wird erfindungsgemäß das CCoAMT-Gen bzw. dessen codierende Region bevorzugt, das der cDNA entspricht, welche auf dem Plasmid pL2-4 liegt. Das Gen bzw. die codierende Region des Gens kann mit Hilfe der cDNA in üblicher Weise erhalten werden.

Der Escherichia coli Stamm DS pL2-4 enthält das Plasmid pL2-4. Dieser Stamm wurde bei der Deutschen Sammlung von Mikroorganismen (DSM), Mascheroder Weg 1b, D-3300 Braunschweig, Bundesrepublik Deutschland in Übereinstimmung mit den Bestimmungen des Budapester Vertrages über die internationale Anerkennung der Hinterlegung von Mikroorganismen für die Zwecke von Patentverfahren hinterlegt (Hinterlegungsdatum: 28. Mai 1991). Er erhielt die Hinterlegungsnummer DSM 6536.

Dieser Stamm sowie seine Mutanten sind ebenfalls Teil der vorliegenden Erfindung. Das in diesem Wirt hinterlegte Plasmid pL2-4 kann in üblicher Weise durch die Vermehrung des Stammes und anschließende Isolierung des Plasmides leicht in den benötigten Mengen gewonnen werden.

Funktionell vollständige Gene, wie die erfindungsgemäßen CCoAMT-Gene, bestehen aus einem regulatorisch wirkenden Teil (insbesondere Promotor) und dem Strukturgen, welches das Protein CCoAMT kodiert.

Beide Genteile können unabhängig voneinander verwendet werden. So ist es möglich, dem regulativ wirkenden Teil eine (vom CCoAMT-Gen abweichende) andere DNA-Sequenz nachzuschalten, welche nach dem Einbau in das Pflanzengenom exprimiert werden soll. Da nur wenige isolierte Promotoren bekannt sind, welche ihre Wirkung in Pflanzen bzw. Pflanzenzellen entfalten können, stellen die Promotoren der CCoAMT-Gene, welche ebenfalls Bestandteile der vorliegenden Erfindung sind, wertvolle Hilfsmittel bei der Erzeugung transformierter Pflanzen bzw. Pflanzenzellen dar.

Ebenso ist es möglich, den CCoAMT-Struktur-Genen einen "fremden" regulatorisch wirkenden Teil vorzuschalten. Dies könnte vorteilhaft sein, wenn bei bestimmten Pflanzen nur bestimmte (z.B. pflanzeneigene) regulatorisch wirkende Gene ausreichend wirksam werden können Die CCoAMT-Struktur-Gene stellen somit wertvolle, selbständig einsetzbare Einheiten dar und sind, wie bereits dargelegt, ebenfalls Teil der vorliegenden Erfindung. Die erfindungsgemäßen CCoAMT-Gene können nach den üblichen Methoden in die regulatorisch wirkenden Teile und die Struktur-Gene getrennt werden. Es ist auch möglich, Teile von verschiedenen natürlich vorkommenden CCoAMT-Genen zu neuen funktionellen "synthetischen" Genen zu kombinieren. Bevorzugt werden die vollständigen natürlichen erfindungsgemäßen CCoAMT-Gene (bzw. die Gen-Einheiten) verwendet. Erfindungsgemäß bevorzugt wird das CCoAMT-Struktur-Gen, welches der cDNA entspricht, die im Plasmid pL2-4 enthalten ist.

Mit Hilfe der üblichen Methoden ist es möglich, die CCoAMT-Gene (bzw. die Gen-Einheiten) oder ihre Teile ein oder mehrfach (z.B. Tandemanordnung), vorzugsweise einfach, in beliebige prokaryontische (vorzugsweise bakterielle) oder eukaryontische (vorzugsweise pflanzliche) DNA als "fremde" oder "zusätzliche" DNA einzubauen. So kann z.B. die der cDNA entsprechende proteincodierende DNA mit regulatorischen Sequenzen versehen werden und in Pflanzen eingebaut werden. Die so "modifizierte" rekombinante DNA, welche z.B. zur Transformation von Pflanzen bzw. Pflanzenzellen verwendet werden kann und nach der Transformation in Pflanzen bzw. Pflanzenzellen enthalten ist, ist Bestandteil der vorliegenden Erfindung.

Die CCoAMT-Gene (bzw. die Gen-Einheiten) und/oder ihre Teile sowie die rekombinante DNA können als "fremde" oder "zusätzliche" DNA in Vektoren (insbesondere Plasmiden, Cosmiden oder Phagen), in transformierten Mikroorganismen (vorzugsweise Bakterien, insbesondere Gramnegativen Bakterien, wie E. coli) sowie in transformierten Pflanzenzellen und Pflanzen bzw. in deren DNA enthalten sein. Solche Vektoren, transformierte Mikroorganismen (die auch diese Vektoren enthalten können) sowie die transformierten Pflanzenzellen und Pflanzen und deren DNA stellen Bestandteile der vorliegenden Erfindung dar.

Wie bereits angedeutet, werden erfindungsgemäß die CCoAMT-Gene (bzw. die Gen-Einheiten) ein- oder mehrfach (an gleichen oder verschiedenen Stellen des Genoms) in das natürliche pflanzliche Genom eingebaut, wobei verschiedene Gene auch mit einander kombiniert werden können. Bei Pflanzen, welche bereits über die Fähigkeit der CCoAMT-Synthese verfügen, kann der Einbau eines oder mehrerer erfindungsgemäßer CCoAMT-Gene zu einem erheblich verbesserten Resistenzverhalten führen. Bei Pflanzen, die keine CCoAMT-Gene enthalten, wird durch den Einbau solcher Gene ebenfalls eine erhöhte Schädlingsresistenz erreicht. Gegebenenfalls werden nur die erfindungsgemäßen Strukturgene verwendet, wobei ein evtl. aus der jeweiligen Pflanze isoliertes regulatorisches DNA Element vorgeschaltet wird.

Die erhöhte Resistenz der erfindungsgemäßen transformierten Pflanzenzellen und Pflanzen ist von Bedeutung für Landwirtschaft und Forsten, für den Zierpflanzenanbau, den Heilpflanzenanbau und die Pflanzenzucht. Auch bei der Kultivierung von Pflanzenzellen, z.B. zur Gewinnung von pharmazeutisch brauchbaren Stoffen, ist es von Vorteil, Pflanzenzellen verfügbar zu haben, welche gegen den Befall durch mikrobielle Schädlinge, insbesondere Pilze, erhöhte Resistenzen aufweisen.

Die vorliegende Erfindung betrifft somit auch ein Verfahren zur Herstellung transformierter Pflanzenzellen (einschließlich Protoplasten) und Pflanzen (einschließlich Pflanzenteile und Samen) mit erhöhter Resistenz gegen Schädlinge, welches dadurch gekennzeichnet ist, daß man

(a) ein oder mehrere CCoAMT-Gene (bzw. Gen-Einheiten) und/oder Teile der CCoAMT -Gene (bzw. der Gen-Einheiten) und/oder erfindungsgemäße rekombinante DNA in den Genom von Pflanzenzellen (einschließlich Protoplasten) einsetzt und gegebenenfalls

(b) aus den transformierten Pflanzenzellen (einschließlich Protoplasten) vollständige transformierte Pflanzen regeneriert und gegebenenfalls vermehrt und gegebenenfalls

(c) von den so erhaltenen transformierten Pflanzen der Elterngeneration oder weiterer daraus gewonnener Generationen die gewünschten Pflanzenteile (einschließlich Samen) gewinnt.

Die Verfahrensschritte (a), (b) und (c) können nach bekannten Verfahren und Methoden in üblicher Weise durchgeführt werden,

4

Transformierte Pflanzenzellen (einschließlich Protoplasten) und Pflanzen (einschließlich Pflanzenteile und Samen), welche ein oder mehrere CCoAMT-Gene (bzw. Gen-Einheiten) und/oder Teile der CCoAMT -Gene (bzw. der Gen-Einheiten) als "fremde" oder "zusätzliche" DNA enthalten sowie solche transformierte Pflanzenzellen und Pflanzen, welche nach den obigen Verfahren erhältlich sind, gehören ebenfalls zur vorliegenden Erfindung.

Teile der vorliegenden Erfindung sind auch die:

(a) Verwendung der CCoAMT-Gene (bzw. der Gen-Einheiten) und/oder ihrer Teile und/oder der erfindungsgemäßen rekombinanten DNA und/oder der erfindungsgemäßen rekombinanten Vektoren und/ oder der erfindungsgemäßen transformierten Mikroorganismen zur Transformation von Pflanzenzellen (einschließlich Protoplasten) und Pflanzen (einschließlich Pflanzenteilen und Samen), die

(b) Verwendung der erfindungsgemäßen transformierten Pflanzenzellen (einschließlich Protoplasten) und Pflanzen (einschließlich Pflanzenteilen und Samen) zur Erzeugung von Vermehrungsmaterial sowie zur Erzeugung neuer Pflanzen und deren Vermehrungsmaterial, die

(c) Verwendung der erfindungsgemäßen CCoAMT-Gene (bzw. der Gen-Einheiten) und/oder ihrer Teile und/oder der erfindungsgemäßen rekombinanten DNA zur Bekämpfung von Schädlingen sowie die

d) Verwendung der auf dem Plasmid pL2-4 enthaltenen cDNA oder ihrer Teile sowie der den Sequenzinformationen gemäß Sequenzprotokoll SEQ ID NO:1 entsprechenden DNA Sequenzen zur Isolierung von CCoAMT-Genen oder deren Teilen aus Pflanzen sowie zur Bestimmung von CCoAMT-Genen in Pflanzen.

Eine Anzahl verschiedener Methoden steht zur Verfügung, die CCoAMT-Gene bzw. die Gen-Einheiten oder ihre Teile als "fremde" oder "zusätzliche" DNA in das genetische Material von Pflanzen bzw. Pflanzenzellen einzusetzen. Der Gentransfer kann nach den allgemein üblichen bekannten Methoden erfolgen, wobei der Fachmann die jeweils geeignete Methode ohne Schwierigkeiten ermitteln kann.

Das Ti-Plasmid von Agrobacterium tumefaciens steht als besonders günstiger und breit einsetzbarer Vektor zur Übertragung von fremder DNA in Genome dikotyler und monokotyler Pflanzen zur Verfügung. Das genetische Material, welches für CCoAMT kodiert, wird zusammen mit regulatorischen DNA-Sequenzen in die T-DNA von geeigneten Ti-Plasmiden eingesetzt (z.B. Zambryski et al. 1983) und durch Infektion der Pflanze, Infektion von Pflanzenteilen oder Pflanzengeweben, wie z.B. von Blattscheiben, Stengeln, Hypokotylen, Kotyledonen, Meristemen und davon ableitenden Geweben, wie z.B. sekundären Embryonen und Kalli oder durch Kokultur von Protoplasten mit Agrobacterium tumefaciens übertragen.

Eine Alternative ist die Inkubation von gereinigter DNA, die das gewünschte Gen enthält in Pflanzenprotoplasten (z.B. Hain et al., 1985; Krens et al., 1982; Paszkowski et al., 1984) in Gegenwart von Polykationen oder Calziumsalzen und Polyethylenglykol.

Die DNA-Aufnahme kann auch zusätzlich durch ein elektrisches Feld (Elektroporation) begünstigt werden (z.B. Fromm et al. 1986).

Die DNA kann in bekannter Weise auch über Pflanzenpollen eingeführt werden, indem Pollen mit physikalisch beschleunigten Partikeln "beschossen" werden, welche die DNA tragen (vgl. EP-A 0 270 356).

Die Regeneration der Pflanzen erfolgt in bekannter Weise mit Hilfe geeigneter Nährmedien (z.B. Nagy und Maliga 1976).

In einer bevorzugten Ausführungsform des erfindungsgemäßen Verfahrens wird die cDNA aus dem Plasmid pL2-4 in einen Expressionsvektor kloniert (z.B. pRT101, Töpfer et.al. 1988). Das chimäre Genkonstrukt wird dann mit dem Restriktionsenzym Hind III isoliert und in einem intermediären Vektor (z.B. pCV001, Koncz und Schell 1986) auf Agrobakterium tumefaciens (Koncz und Schell 1986) übertragen.

Alternativ wird das chimäre Genkonstrukt in die HindIII-Stelle des Plasmido PIGVneo 1103 (Hain et.al. 1985) kloniert, und in einer besonders bevorzugten Ausführungsform wird das chimäre Genkonstrukt im Plasmid pLGVneo 1103 in üblicher weise durch direkten Gentransfer auf Pflanzenprotoplasten übertragen (z.B. Hain et.al. 1985). Dabei kann das Plasmid in zirkulärer, vorzugsweise jedoch in linearer Form, vorliegen.

Bei der Verwendung dieses Plasmids mit Reportergen werden Kanamycin-resistente Protoplasten dann auf Expression von CCoAMT überprüft.

Transformierte (transgene) Pflanzen bzw. Pflanzenzellen werden nach den bekannten Methoden, z.B. durch Blattscheiben Transformation (z.B. Horsch et al. 1985) durch Cokultur regenerierender Pflanzenprotoplasten oder Zellkulturen mit Agrobacterium tumefaciens (z.B. Marton et al. 1979, Hain et al. 1985) oder durch direkte DNA Transfektion erzeugt. Resultierende transformierte Pflanzen werden entweder durch Selektion auf die Expression des Reportergens, z.B. durch die Phosphorylierung von Kanamycinsulfat in vitro (Reiss et al. 1984; Schreier et al. 1985) oder durch die Expression der Nopalinsynthase (nach Aerts et al. 1983) oder von CCoAMT durch Northern-Blot-Analyse und Western Blot-Analyse nachgewiesen. Die CCoAMT kann auch in bekannter Weise mit Hilfe spezifischer Antikörper in transformierten Pflanzen

5

nachgewiesen werden.

Die Kultivierung der transformierten Pflanzenzellen sowie die Regeneration zu vollständigen Pflanzen erfolgt nach den allgemein üblichen Methoden mit Hilfe der jeweils geeigneten Nährmedien.

Sowohl die transformierten Pflanzenzellen als auch die transformierten Pflanzen, welche die erfindungsgemäßen CCoAMT-Gene (bzw. die Gen-Einheiten) enthalten und welche Bestandteile der vorliegenden Erfindung sind, zeigen eine erheblich höhere Resistenz gegen Schädlinge, insbesondere pflanzenpathogene Pilze.

Im Zusammenhang mit der vorliegenden Erfindung bedeutet der Ausdruck "Pflanzen" sowohl vollständige Pflanzen als auch Pflanzenteile, wie Blätter, Samen, Knollen, Stecklinge u.s.w. "Pflanzenzellen" schließen Protoplasten, Zelllinien, Pflanzenkalli usw. ein. "Vermehrungsmaterial" bedeutet Pflanzen und Pflanzenzellen, welche zur Vermehrung der transformierten Pflanzen und Pflanzenzellen verwendet werden können und ist somit ebenfalls Teil der vorliegenden Erfindung.

Im vorliegenden Zusammenhang bedeutet der Ausdruck "im wesentlichen gleichwirkende DNA-Sequenzen", daß die Erfindung auch solche Modifikationen umfaßt, bei welchen die Funktion der CCoAMT-Gene und ihrer Teile nicht derart beeinträchtigt ist, daß CCoAMT nicht mehr gebildet wird oder der regulatorische Genteil nicht mehr wirksam wird. Entsprechende Modifikationen können durch den Ersatz, die Hinzufügung und/oder die Entfernung von DNA-Abschnitten, einzelner Kodons und/oder einzelner Nucleotide erfolgen.

Bei den erfindungsgemäß verwendbaren Mikroorganismen bedeutet "Mutanten" solche modifizierten Mikroorganismen, welche noch die für die Ausführung der Erfindung wesentlichen Merkmale aufweisen, insbesondere die jeweiligen Plasmide enthalten.

Zu den Pflanzen, welchen durch den Einbau (Transformation) der erfindungsgemäßen CCoAMT-Gene (bzw. der Gen-Einheiten) Resistenz bzw. eine erhöhte Resistenz gegenüber den Schädlingen verliehen werden kann, gehören praktisch alle Pflanzen. Ein besonderes Bedürfnis zur Resistenzerzeugung besteht naturgemäß bei den Kulturpflanzen, wie Forstpflanzen, z.B. Fichten, Tannen, Douglasien, Kiefern, Lärchen, Buchen und Eichen sowie Nahrungsmittel und Rohstoffe liefernden Pflanzen, z.B. Getreide (insbesondere Weizen, Roggen, Gerste, Hafer, Hirse, Reis und Mais), Kartoffel, Leguminosen (wie Hülsenfrüchte und insbesondere Alfalfa, Sojabohnen), Gemüse (insbesondere Kohlarten und Tomaten), Obst (insbesondere Äpfel, Birnen, Kirschen, Weintrauben, Citrus, Ananas und Bananen), Ölpalmen, Tee-, Kakao- und Kaffeesträucher, Tabak, Sisal und Baumwolle sowie bei Heilpflanzen, wie Rauwolfia und Digitalis. Besonders bevorzugt seien Kartoffel, Tomaten und Leguminosen genannt. Vorzugsweise werden die erfindungsgemäßen CCoAMT-Gene als "fremde" DNA in den Genom von Pflanzen eingebaut.

Als Schädlinge, gegen welche mit Hilfe der erfindungsgemäßen CCoAMT-Gene Resistenzen, bzw. erhöhte Resistenzen erzielt werden können, seien tierische Schädlinge, wie Insekten, Milben und Nematoden sowie mikrobielle Schädlinge, wie phytopathogene Pilze, Bakterien und Viren genannt. Besonders hervorgehoben werden mikrobielle Schädlinge, insbesondere phytopathogene Pilze.

Zu den schädlichen Insekten gehören insbesondere Insekten der Ordnungen:

Orthoptera, Dermaptera, Isoptera, Thysanoptera, Heteroptera, Homoptera, Lepidoptera, Coleoptera, Hymenoptera und Diptera.

Zu den schädlichen Milben gehören insbesondere: Tarsonemus spp., Panonychus spp. und Tetranychus spp.

Zu den schädlichen Nematoden gehören insbesondere: Pratylenchus spp., Heterodera spp. und Meloidogyne spp.

Zu den mikrobiellen Schädlingen gehören insbesondere die phytopathogenen Pilze:

Plasmodiophoromycetes, Oomycetes, Chytridiomycetes, Zygomycetes, Ascomycetes, Basidiomycetes, Deuteromycetes.

Zu den phytopathogenen Bakterien gehören insbesondere die Pseudomonadaceae, Rhizobiaceae, Enterobacteriaceae, Corynebacteriaceae und Streptomycetaceae.

Zu den Viruserkankungen gehören insbesondere Mosaik-, Verzwergungs- und Vergilbungsvirosen.

Beispielhaft aber nicht begrenzend seien einige Erreger von virösen, pilzlichen und bakteriellen Erkrankungen, die unter die oben aufgezählten Oberbegriffe fallen, genannt:

Barley Yellow Dwarf Virus (BYDV), Potato Virus Y (PVY), Cucumber Mosaic Virus (CMV), Watermelon Mosaic Virus (WMV), Tristeza-Virus, Tobacco Mosaic Virus (TMV), Tobacco Necrosis Virus (TNV), Beet necrotic Yellow Vein Virus (BNYVV), Rhizomania-Virus.

Xanthomonas-Arten, wie beispielsweise Xanthomonas campestris pv. oryzae;

Pseudomonas-Arten, wie beispielsweise Pseudomonas syringae pv. lachrymans;

Erwinia-Arten, wie beispielsweise Erwinia amylovora;

Pythium-Arten, wie beispielsweise Pythium ultimum;

Phytophthora-Arten, wie beispielsweise Phytophthora infestans;

Pseudoperonospora-Arten, wie beispielsweise Pseudoperonospora humuli oder Pseudoperonospora cubense;

Plasmopara-Arten, wie beispielsweise Plasmopara viticola;

Peronospora-Arten, wie beispielsweise Peronospora pisi oder P. brassicae;

Erysiphe-Arten, wie beispielsweise Erysiphe graminis;

Sphaerotheca-Arten, wie beispielsweise Sphaerotheca fuliginea;

Podosphaera-Arten, wie beispielsweise Podosphaera leucotricha;

Venturia-Arten, wie beispielsweise Venturia inaequalis;

Pyrenophora-Arten, wie beispielsweise Pyrenophora teres oder P. graminea (Konidienform: Drechslera, Syn: Helminthosporium);

Cochliobolus-Arten, wie beispielsweise Cochliobolus sativus (Konidienform: Drechslera, Syn: Helminthosporium);

Uromyces-Arten, wie beispielsweise Uromyces appendiculatus;

Puccinia-Arten, wie beispielsweise Puccinia recondita;

Tilletia-Arten, wie beispielsweise Tilletia caries;

Ustilago-Arten, wie beispielsweise Ustilago nuda oder Ustilago avenae;

Pellicularia-Arten, wie beispielsweise Pellicularia sasakii;

Pyricularia-Arten, wie beispielsweise Pyricularia oryzae;

Fusarium-Arten, wie beispielsweise Fusarium culmorum;

Botrytis-Arten, wie beispielsweise Botrytis cinerea;

Septoria-Arten, wie beispielsweise Septoria nodorum;

Leptosphaeria-Arten, wie beispielsweise Leptosphaeria nodorum;

Cercospora-Arten, wie beispielsweise Cercospora canescens;

Alternaria-Arten, wie beispielsweise Alternaria brassicae;

Pseudocercosporella-Arten, wie beispielweise Pseudocerco sporella herpotrichoides. Weiterhin sei Helminthosporium carbonum aufgeführt.

Die vorliegende Erfindung soll anhand der folgenden beispielhaften Ausführungen näher erläutert werden:

## 1. Isolierung des Gens für CCoAMT aus Petersilie

Pflanzen und Zellkulturen aus Petersilie (Petroselinum crispum) enthalten Gene für CCoAMT, welche die Bildung von CCoMAT (Größe des Proteins 27 000 D; Reaktion mit spezifischem Antiserum) bewirken.

Bei der Isolierung der CCoAMT-Gene wurden die bekannten Verfahren und Methoden der Molekularbiologie verwendet, wie sie beispielsweise in folgendem Handbuch detailliert beschrieben werden: Maniatis, T., Fritsch, E.F., Sambrook, J.: Molecular Cloning: A Laboratory Manual; Cold Spring Harbor Laboratory, Second Edition 1989.

Es wird zunächst eine "Gen-Bibliothek" für Petersilie angelegt: Genomische DNA aus angereicherten Zellkernen (Bedbrook, J., Plant Molecular Biology Newsletter 2, 24, 1981) wird mit dem Restriktions-Enzym NdeII so geschnitten, daß DNA-Fragmente mit einer Durchschnittslänge von etwa 12 000 Nukleotidpaaren entstehen. Diese Fragmente werden in die BamHI-Stelle von Lambda-Phage EMBL4 kloniert (Frischauf et al., J. Mol. Biol. 170, 827-842, 1983), und die Phagen werden in E. coli vermehrt. Die Gesamtheit der Phagen-Population enthält, kloniert in Teilstücken, die gesamte genomische DNA der Petersilie, und damit auch die Gene für CCoMAT.

Die Gene für CCoMAT, ihre mRNA und die CCoMAT-Synthasen-cDNA enthalten jeweils gleiche Nucleinsäuresequenzen, da sie voneinander abgeleitet werden können (Gen→ mRNA→cDNA). Dies bedeutet, daß die Gene für CCoMAT durch spezifische Hybridisierung mit CCoMAT-cDNA (vergl. SEQ ID NO:1) bzw. mit spezifischen Oligonukleotiden, die aus dieser Sequenz ableitbar sind, identifizierbar sind. Die Phagen mit den Genen werden durch Hybridisierung identifiziert, dann isoliert und vermehrt. Die in diesem Phagen klonierte genomische DNA aus Petersilie wird weiter durch Analyse mit verschiedenen Restriktionsenzymen kartiert, und die Position der CCoMAT-Gene wird durch weitere Hybridisierungs-Experimente mit cDNA-Sequenzen bzw. synthetischen Oligonukleotiden festgelegt. Schließlich werden die Gen-Einheiten durch Verdau mit Restriktionsenzymen aus dem Phagen herausgeschnitten, im entsprechend geschnittenen Plasmid-Vektor kloniert und als rekombinante Plasmide vermehrt.

Zur Isolierung weiterer erfindungsgemäßer CCoAMT-Gene können auf Grund der Sequenzhomologien DNA-Sequenzen als Sonden verwendet werden, welche den Sequenzen entsprechen, die in den auf den Plasmid pL2-4 befindlichen cDNA enthalten sind, Auch die ganze cDNA des Plasmids kann hierfür verwendet werden.

7

2. Transformation von Tabak

a) Kultur von Tabaksprossen und Isolierung von Tabakprotoplasten:

Nicotiana tabacum (Petit Havanna SR1) wird als sterile Sproßkultur auf hormonfreiem LS Medium (Linsmaier und Skoog 1965) vermehrt. In Abständen von ca. 6-8 Wochen werden Sproßabschnitte auf frisches LS-Medium umgesetzt. Die Sproßkulturen werden bei 12 h Licht (1000-3000 Lux) in einem Kulturraum bei 24-26°C gehalten.

Für die Isolierung von Blattprotoplasten werden ca. 2 g Blätter (ca. 3-5 cm lang) mit einer frischen Rasierklinge in kleine Stücke (0,5 cm x 1 cm) geschnitten. Das Blattmaterial wird in 20 ml Enzymlösung, bestehend aus K3 Medium (Nagy und Maliga 1976), 0,4 m Saccharose, pH 5,6, 2 % Zellulase R10 (Serva), 0,5 % Macerozym R10 (Serva) für 14-16 h bei Raumtemperatur inkubiert. Danach werden die Protoplasten durch Filtration über 0,30 mm und 0,1 mm Stahlsiebe von Zellresten getrennt. Das Filtrat wird 10 Minuten lang bei 100 x g zentrifugiert. Während dieser Zentrifugation flotieren intakte Protoplasten und sammeln sich in einer Bande am oberen Rand der Enzymlösung. Das Pellet aus Zellresten und die Enzymlösung werden mit einer Glaskapillare abgesaugt. Die vorgereinigten Protoplasten werden mit frischem K3 Medium (0,4 M Saccharose als Osmotikum) auf 10 ml aufgefüllt und erneut flotiert. Das Waschmedium wird abgesaugt und die Protoplasten werden für Kultur oder folgende Infektion mit Agrobakterien (Kokultur) auf 1-2 x $10^5$/ml verdünnt. Die Protoplastenkonzentration wird in einer Zählkammer bestimmt.

b) Konstruktion eines Chimären CCoAMT-Gens und Übertragung in Agrobacterium tumefaciens

Das EcoRI Fragment aus pL2-4 (ca. 1,2 kb) wird in die EcoRI-Stelle des Vektors pRT 101 (Töpfer et.al. 1988) kloniert. Hierdurch erhält die cDNA an ihrem 5' Ende den 35 S Promotor von CaMV und an ihrem 3' Ende eine Polyadenylierungssequenz aus CaMV. Dieses chimäre Genkonstrukt kann dann durch Spaltung mit HindIII funktionell isoliert werden als ein Fragment von ca. 1,9 kb. Dieses HindIII Fragment kann dann mit den üblichen Methoden in einen intermediaren Vektor, z.B. pCV001 (Koncz und Schell, 1986) übertragen werden. Statt der genannten Vektoren können beliebige andere Expressionsvektoren und intermediäre Vektoren, die entsprechende Schnittstellen aufweisen, eingesetzt werden, wobei der Fachmann anhand der obigen Angaben eine geeignete Auswahl leicht treffen kann. Der resultierende intermediäre Vektor, der das CCoAMT-Gen enthält, wird nach Agrobacterium tumefaciens, das eine funktionelle vir-Region enthält, übertragen (Koncz und Schell 1986, van Haute et.al. 1983).

c) Transformation von regenerierenden Tabakprotoplasten durch Kokultur mit Agrobacterium tumefaciens:

Es wird im folgenden die Methode von Marton et al. 1979 mit kleinen Veränderungen benutzt. Die Protoplasten werden wie beschrieben isoliert und in einer Dichte von 1-2 x $10^5$/ml in K3 Medium (0,4 m Saccharose, 0,1 mg/l NAA, 0,2 ml in K3 Medium (0,4 m Saccharose, 0,1 mg/l NAA, 0,2 mg Kinetin) 2 Tage im Dunkeln und ein bis zwei Tage lang unter Schwachlicht (500 lux) bei 26°C inkubiert, Sobald die ersten Teilungen der Protoplasten auftreten, werden 30 $\mu$l einer Agrobakteriumsuspension gemäß b) in minimal A (Am) Medium (Dichte ca. $10^9$ Agrobakterien/ ml) zu 3 ml regenerierenden Protoplasten gegeben. Die Kokulturdauer beträgt 3-4 Tage bei 20°C im Dunkeln, Danach werden die Tabakzellen in 12 ml Zentrifugenröhrchen gefüllt, mit Seewasser (600 mOsm/kg) auf 10 ml verdünnt und bei 60 x g 10 Minuten lang pelletiert. Dieser Waschvorgang wird noch 1-2 x wiederholt um den größten Teil der Agrobakterien zu entfernen. Die Zellsuspension wird in einer Dichte von 5 x $10^4$/ml in K3 Medium (0,3 m Saccharose) mit 1 mg/1 NAA (Naphthyl-1-essigsäure), 0,2 mg/l Kinetin und 500 mg/l des Cephalosporin-Antibiotikums Cefotaxim kultiviert. Die Zellsuspension wird jede Woche mit frischem K3 Medium verdünnt und der osmotische Wert des Mediums graduell um 0,05 m Saccharose (ca. 60 mOsm/kg) pro Woche reduziert. Die Selektion mit Kanamycin (100 mg/l Kanamycinsulfat (Sigma), 660 mg/g aktives Km) wird 2-3 Wochen nach der Kokultur in Agarose "bead type culture" (Shillito et al. 1983) gestartet. Kanamycinresistente Kolonien können 3-4 Wochen nach Beginn der Selektion vom Hintergrund zurückgebliebender Kolonien unterschieden werden.

d) Direkte Transformation von Tabakprotoplasten mit DNA. Calciumnitrat-PEG Transformation.

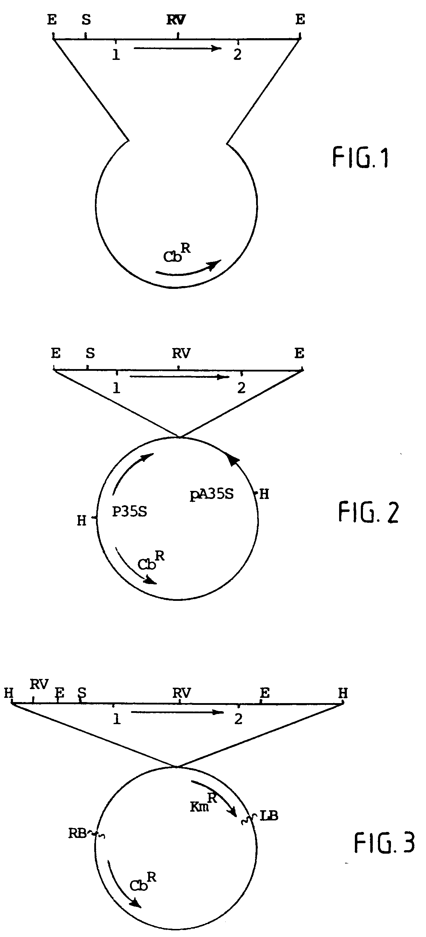

In einer Petrischale werden ca. $10^6$ Protoplasten in 180 $\mu$l K3 Medium mit 20 $\mu$l wäßriger DNA Lösung welche 20 $\mu$g Plasmid pCV001::CCoAMT (vgl. Fig. 3) enthält, vorsichtig gemischt. Das Plasmid pCV001::CCoAMT ist nach bekannten Methoden aus dem Plasmid pCV001, pRT101 und pL2-4 erhältlich (vgl. Fig. 1-3). Anschließend werden 200 $\mu$l Fusionslösung (0,1 m Calciumnitrat, 0,45 M Mannit, 25 % Polyethylenglykol (PEG 6000), pH 9) vorsichtig zugegeben. Nach 15 Minuten werden 5 ml Waschlösung (0,275 M Calciumnitrat pH 6) addiert und nach weiteren 5 Minuten werden die Protoplasten in ein Zentrifugenröhrchen transferiert und bei 60 x g pelliert. Das Pellet wird in einer kleinen Menge K3 Medium aufgenommen und wie im nächsten Abschnitt beschrieben kultiviert. Alternativ können die Protoplasten nach Hein et al. 1985 transformiert werden.

e) Kultur der mit DNA inkubierten Protoplasten und Selektion Kanamycin resistenter Kalli:

Für die im folgenden beschriebene Kultur und Selektion Kanamycin resistenter Kolonien wird eine modifizierte "Bead Type culture"-Technik (Shillito et al. 1983) verwendet. Eine Woche nach Behandlung der Protoplasten mit DNA (vgl. d) werden 3 ml der Zellsuspension mit 3 ml K3 Medium (0,3 M Saccharose + Hormone; 1,2 % (Seaplaque) LMT Agarose (low melting agarose, Marine Colloids) in 5 cm Petrischalen gemischt. Für diesen Zweck wird Agarose trocken autoklaviert und nach Zugabe von K3 Medium im Mikrowellenherd kurz aufgekocht. Nach Erstarren der Agarose werden die Agarosescheiben ("beads") mit den eingebetteten Tabakmikrokalli für weitere Kultur und Selektion in 10 cm Petrischalen transferiert und je 10 ml K3 Medium (0,3 M Saccharose, 1 mg/l NAA, 0,2 mg/l Kinetin) und 100 mg/l Kanamycinsulfat (Sigma) addiert. Das Flüssigmedium wird jede Woche gewechselt. Dabei wird der osmotische Wert des Mediums stufenweise herabgesetzt.

Pro Woche wird das Austauschmedium (K3 + Km) um 0,05 m an Saccharose (ca. 60 mOsm) reduziert.

Schema der Selektion kanamycinresistenter Tabakkolonien nach DNA Transformation:

```
   0,4 M   0,3 M   0,25 M   0,20 M   0,15M   0,10 M   Saccha-

                                                      rose im

                                                      Flüssig-

                                                      medium

   A   E S                                    K
   _____

       1       2        3        4       5        6   Wochen

                                                      nach DNA

                                                      Aufnahme

       (K3 Medium 1 mg NAA, 0,2 mg Kinetin)


   A = DNA Aufnahme

   E = Einbettung in Agarose

   S = Selektion mit Kanamycin (100 mg/l Kanamycinsulfat)

   K = Kanamycinresistente Kolonien können vom Hintergrund

       eindeutig unterschieden werden
```

e) Regeneration kanamycinresistenter Pflanzen:

Sobald die kanamycinresistenten Kolonien einen Durchmesser von ca. 0,5 cm erreicht haben, wird die Hälfte auf Regenerationsmedium (LS-Medium, 2 % Saccharose, 0,5 mg/l Benzylaminopurin BAP) gesetzt und bei 12 h Licht (3000-5000 lux) und 24°C im Kulturraum gehalten. Die andere Hälfte wird als Kalluskultur auf LS Medium mit 1 mg/l NAA, 0,2 mg/l Kinetin, 0,1 mg/l BAP und 100 mg/l Kanamycinsulfat propagiert. Wenn die regenerierten Sproße ca. 1 cm groß sind, werden sie abgeschnitten und auf 1/2 LS Medium (1 % Saccharose, 0,8 % Agar) ohne Wachstumsregulatoren zur Bewurzelung gesetzt. Die Sproße werden auf 1/2 MS-Medium mit 100 mg/l Kanamycinsulfat bewurzelt und später in Erde umgesetzt.

g) Transformation von Blattscheiben durch Agrobacterium tumefaciens

Für die Transformation von Blattscheiben (Horsch et al. 1985) werden ca. 2-3 cm lange Blätter von sterilen Sproßkulturen in Scheiben von 1 cm Durchmesser gestanzt und mit einer Suspension entsprechender Agrobacterien (ca. $10^9$/ml) (vgl. c) in Am-Medium, siehen unten) für ca. 5 Minuten inkubiert. Die infizierten Blattstücke werden auf MS-Medium (siehe unten) ohne Hormone für 3-4 Tage bei ca. 24°C gehalten. Während dieser Zeit überwächst Agrobakterium die Blattstücke. Die Blattstücke werden anschließend in MS-Medium (0,5 mg/ml BAP, 0,1 mg/ml NAA) gewaschen und auf das gleiche Medium (0,8 % Agar) mit 500 $\mu$g/ml Cefotaxim und 100 $\mu$g/ml Kanamycinsulfat gelegt. Nach zwei Wochen sollte das Medium erneuert werden, Transformierte Kanamycinresistente Sproße werden nach weiteren 2-3 Wochen sichtbar.

Biochemische Nachweismethode der Transformation

Neomycin-Phosphotransferase (NPT II) Enzymtest:

NPT II Aktivität in Pflanzengewebe wird durch in situ Phosphorylierung von Kanamycin, wie bei Reiß et al. (1984) beschrieben und von Schreier et al. (1985) modifiziert, wie folgt, nachgewiesen. 50 mg Pflanzengewebe wurden in 50 $\mu$l Extraktionspuffer (10 % Glycerin, 5 % 2-Mercaptoethanol, 0,1 % SDS, 0,025 % Bromphenolblau, 62,5 mM Tris pH 6,8) unter Zusatz von Glaspulver auf Eis homogenisiert und 10 Minuten lang in einer Eppendorfzentrifuge bei 4°C zentrifugiert. 50 $\mu$l des Überstandes werden auf ein natives Polyacrylamidgel (145 x 110 x 1,2 mm; Trenngel: 10 % Acrylamid, 0,33 % Bisacrylamid, 0,375 M Tris pH 8,8, Sammelgel: 5 % Acrylamid, 0,165 % Bisacrylamid, 0,125 M Tris pH 6,8) aufgetragen und über Nacht bei 4°C und 60 V elektrophoretisiert. Sobald der Bromphenolblau-Marker aus dem Gel herausläuft, wird das Gel zweimal mit destilliertem Wasser 10 Min. lang und einmal 30 Min. mit Reaktionspuffer gewaschen (67 mM Tris-Maleat, pH 7,1, 42 mM MgCl$_2$, 400 mM Ammoniumchlorid). Das Gel wird auf eine gleichgroße Glasplatte gelegt und mit 40 ml 1 %iger Agarose in Reaktionspuffer, der die Substrate Kanamycinsulfat (20 $\mu$g/ml) und 20-200 $\mu$Ci $^{32}$P ATP (Amersham) enthält, überschichtet. Das Sandwichgel wird 30 Min. bei Zimmertempreratur inkubiert und dann wird ein Blatt Phosphozellulosepapier P81 (Whatman) auf die Agarose gelegt. Darüber werden vier Filtrierpapierlagen 3 MM, (Whatman) und einige Papierhandtücher gestapelt. Der Transfer von in situ phosphoryliertem radioaktiven Kanamycinphosphat auf das P81 Papier wird nach 3-4 h gestoppt. Das P81 Papier wird für 30 min. in einer Lösung von Proteinase K und 1 % Natriumdodecyl sulfat (SDS) bei 60°C inkubiert und dann 3-4 mal in 250 ml 10 mM Phosphatpuffer pH 7,5 bei 80°C gewaschen, getrocknet und für 1-12 h lang bei -70°C autoradiografiert (XAR5 Film Kodak).

4. Transformation von Solanum tuberosum (Kartoffel)

Die Transformation wurde genau nach dem in der EP-A-0 242 246, Seiten 14 bis 15 angegebenen Weise vorgenommen, wobei die Agrobakterien Ti-Plasmide enthalten, die das CCoAMT-Gen oder die CCoAMT-Gene tragen.

Alle Prozentangaben in den obigen Beispielen beziehen sich auf Gewichtsprozente, wo nichts anderes angegeben wird.

In den gemäß den obigen Beispielen erhaltenen Pflanzenzellen und Pflanzen (Tabak) wurde die Anwesenheit der CCoAMT-Gene durch Southern Blot Analyse bestätigt. Die Expression der CCoAMT-Gene wurde durch Northern Blot Analyse, CCoAMT mit Hilfe von spezifischen Antikörpern nachgewiesen.

Im folgenden werden einige der bei der Transformation von Pflanzen bzw. Pflanzenzellen eingesetzte Medien beschrieben:

| Am-Medium | |
|---|---|
| 3,5 g | K$_2$HPO$_4$ |
| 1,5 g | KH$_2$PO$_4$ |
| 0,5 g | Na$_3$ Citrat |
| 0,1 g | MgSO$_4$ x 7H$_2$O |
| 1 g | (NH$_4$)$_2$SO$_4$ |
| 2 g | Glukose |
| | ad 1 l |

Medium für sterile Sproßkultur von Tabak

Macro-elemente 1/2 der Konzentration der MS Salze
Micro-elemente 1/2 der Konzentration der MS Salze

| Fe-EDTA | | Murashige und Skoog (MS) |
|---|---|---|
| Myo-Inosit | | 100 mg/l |
| Sucrose | | 10 mg/l |
| Agar | | 8 g/l |
| Vitamine | Ca-panthotenat | 1 mg/l |
| | Biotin | 10 mg/l |
| | Nicotinsäure | 1 mg/l |
| | Pyridoxin | 1 mg/l |
| | Thiamin | 1 mg/l |
| pH 5,7 vor dem Autoklavieren | | |

K3-Medium

Zur Kultur von Nicotiana tabacum petit Havana SR1, Nicotiana tabacum Wisconsin 38, und Nicotiana plumaginifolia Protoplasten (Nagy und Maliga, 1976)

| Macro-elemente | $NH_4NO_3$ | 250 mg/l |
|---|---|---|
| | $KNO_3$ | 2500 mg/l |
| | $CaCl_2 \cdot 2H_2O$ | 900 mg/l |
| | $MgSO_4.7H_2O$ | 250 mg/l |
| | $NaH_2PO_4.1H_2O$ | 150 mg/l |
| | $(NH_4)_2SO_4$ | 134 mg/l |
| | $CaHPO_4.1H_2O$ | 50 mg/l |
| Micro-elemente | $H_3BO_3$ | 3 mg/l |
| | $MnSO_4.1H_2O$ | 10 mg/l |
| | $ZnSO_4.4H_2O$ | 2 mg/l |
| | KI | 0,75 mg/l |
| | $Na_2MoO_4.2H_2O$ | 0,25 mg/l |
| | $CuSO_4.5H_2O$ | 0,025 mg/l |
| | $CoCl_2.6H_2O$ | 0,025 mg/l |
| Fe-EDTA | $Na_2EDTA$ | 37,2 mg/l |
| | $FeSO_4.7H_2O$ | 27,8 mg/l |
| Inosit | | 100 mg/l |
| Sucrose | | 137 g/l (= 0,4 M) |
| Xylose | | 250 mg/l |
| Vitamine | Nicotinsäure | 1 mg/l |
| | Pyridoxin | 1 mg/l |
| | Thiamin | 10 mg/l |
| Hormone | NAA | 1,0 mg/l |
| | Kinetin | 0,2 mg/l |
| pH 5,6 | | |
| Filter sterilisieren | | |

Linsmaier und Skoog Medium (Linsmaier und Skoog 1965)

Zur Kultur von regenerierenden Protoplasten und für Gewebekultur von Tabaktumoren und Kallus. Linsmaier und Skoog (LS) Medium ist Murashige und Skoog Medium (Murashige und Skoog, 1962) mit den folgenden Modifikationen:

- Thiamin wird in höherer Konzentration eingewogen 0,4 mg/l anstatt 0,1 mg/l;
- Glycin, Pyridoxin und Nicotinsäure fehlen.

| Macro-elemente | $NH_4NO_3$ | 1650 mg/l |
| | $KNO_3$ | 1900 mg/l |
| | $CaCl_2.2H_2O$ | 440 mg/l |
| | $MgSO_4.7H_2O$ | 370 mg/l |
| | $KH_2PO_4$ | 170 mg/l |
| Micro-elemente | $H_3BO_3$ | 6,2 mg/l |
| | $MnSO_4.1H_2O$ | 22,3 mg/l |
| | $ZnSO_4.4H_2O$ | 8,6 mg/l |
| | KI | 0,83 mg/l |
| | $Na_2MoO_4.2H_2O$ | 0,25 mg/l |
| | $CuSO_4.5H_2O$ | 0,025 mg/l |
| | $CoCl_2.6H_2O$ | 0,025 mg/l |
| Fe-EDTA | $Na_2EDTA$ | 37,2 mg/l |
| | $FeSO_4.7H_2O$ | 27,8 mg/l |
| Inosit | | 100 mg/l |
| Saccharose | | 30 g/l |
| Agar | | 8 g/l |
| Vitamine | Thiamin | 0,4 mg/l |
| Hormone: | NAA | 1 mg/l |
| | Kinetin | 0,2 mg/l |
| pH 5,7 vor dem Autoklavieren | | |

Zur Transformation von Pflanzen bzw. Pflanzenzellen kann die folgende Literatur angeführt werden:

Fraley R.T., Rogers S.G., Horsch R.B., Sanders P.R., Flick J.S., Adams S.P., Bittner M.L., Brand L.A., Fink C.L., Fry J.S., Fallupi G.R., Goldberg S.B., Hoffmann N.L., Woo S.C. (1983). Expression of bacterial genes in plant cells. Proc. Natl. Acad. Sci. USA 80:4803-4807.

Fromm ME, Taylor LP, Walbot V (1986) Stable transformation of maize after gene transfer by electroporation. Nature 319: 791-793

Hain, R., Stabel, P., Czernilofsky, A.P., Steinbiß, H.H., Herrera-Estrella, L., Schell, J. (1985) Uptake, integration, expression and genetic transmission of a selectable chimeric gene by plant protoplasts. Molec Gen Genet 199: 161-168

Hernalsteens JP, Thia-Tong L, Schell J, Van Montagu M (1984) An Agrobacterium-transformed Cell culture from the monocot Asparagus officinalis. EMBO J 3:3039-3041

Herrera-Estrella L., De Block M., Messens E., Hernalsteens JP., van Montagu M., Schell J. (1983) EMBO J. 2: 987-995.

Horsch RB, Fry JE, Hoffmann NL, Eichholtz D, Rogers SG, Fraley RT (1985) A simple and general method for transferring genes into plants. Science 277: 1229-1231 Krens FH, Molendijk L, Wullems GJ, Schilperoort RA (1982) in vitro transformation of plant protoplasts with Ti-plasmid DNA. Nature 296; 72-74

Koncz C, Schell J (1986) The promotor of $T_L$-DNA gene 5 controls the tissue-specific expression of chimaeric genes carried by a noval type of Agrobacterium linary vector. Mol. Gen. Genet. (1986) 204: 338-396

Linsmaier DM, Skoog F (1965) Organic growth factor requirements of tobacco tissue cultures, Physiol Plant 18: 100-127

Marton L, Wullems GJ, Molendijk L, Schilperoort PR (1979) In vitro transformation of cultured cells from Nicotiana tabacum by Agrobacterium tumefaciens. Nature 277: 1229-131

Nagy JI, Maliga P (1976) Callus induction and plant regeneration from mesophyll protoplasts of Nicotiana sylvestris. Z Pflanzenphysiol 78: 453-455

Paszkowski J, Shillito RD, Saul M, Mandak V, Hohn T, Hohn B, Potrykus I (1984) Direct gene transfer to plants. EMBO J 3: 2717-2722

Shillito RD, Paszkowski J. Potrykus I (1983) Agarose plating and Bead type culture technique enable and stimulate development of protoplast-derived colonies in an number of plant species. Pl Cell Rep 2: 244-247 Van den Elzen PJM, Townsend J, Lee KY, Bedbrook JR (1985) A chimaeric resistance gen as a

EP 0 516 958 A2

selectable marker in plant cells. Plant Mol. Biol. 5, 299-302.

Van den Elzen PJM, Townsend J, Lee KY, Bedbrook JR (1985) A chimaeric resistance gen as a selectable marker in plant cells. Plant Mol. Biol. 5, 299-302.

Velten J, Velten L, Hain R, Schell J (1984) Isolation of a dual plant promotor fragment from the Ti Plasmid of Agrobacterium tumefaciens. EMBO J 12: 2723-2730

Van Haute E, Joos H, Maes M, Warren G, Van Montagu M, Schell J (1983) Intergenic transfer and excharge recombination of restriction fragments clones in pBR 322: a novel strategy for the reversed genetics of Ti plasmids of /Agrobacterium tumefacines. EMBO J 2: 411-418.

Zambryski P, Joos H, Genetello C, van Montagu M, Schell J (1983) Ti-plasmid vector for the introduction of DNA into plant cells without altering their normal regeneration capacity, EMBO J 12: 2143-2150.

Reiss, B., Sprengel, Will H., and Schaller H(1984) A new sensitive method for qualitative and quantitative assay of neomycin phosphotransferase in crude cell tracts, GENE 1081: 211-217

Schreier P.H., Seftor E.A., Schall J. and Bohnert H.J. (1985) The use of nuclear-encoded sequences to direct the light-regulated synthesis and transport of a foreingn protein into plant chloroplasts, EMBO J Vol. 4, No. 1: 25-32

Weiterhin können die folgenden veröffentlichten Patentanmeldungen aufgeführt werden:

| | |
|---|---|
| EP-A 116 718 | EP-A-126 546 |
| EP-A 159 418 | EP-A-164 597 |
| EP-A 120 515 | EP-A-175 966 |
| EP-A-120 516 | WO 84/02913 |
| EP-A-172 112 | WO 84/02919 |
| EP-A-140 556 | WO 84/02920 |
| EP-A-174 166 | WO 83/01176 |
| EP-A-122 791 | |

Die erhöhte Resistenz der erfindungsgemäßen transformierten Pflanzen sei anhand des bolgenden Beispiels erläutert:

Nachweis der erhöhten Resistenz von transformierten Pflanzen

Beispiel A

Zur Prüfung einer erhöhten Resistenz gegenüber Pflanzenkrankheiten werden die Pflanzen mit einem Pathogen inokuliert und der Befallsgrad als Parameter herangezogen. Als Testpathogen dient Botrytis cinerea Pers..

Die Tabakpflanzen werden in Gewebekultur vorgezogen und anschließend im Gewächshaus in Einheitserde (Fa. Balster) in Töpfe (d = 11 cm) getopft und bei 23°C und 70-80 % rel. Luftfeuchte im Gewächshaus bis Versuchsbeginn angezogen. Die Versorgung mit Wasser und Dünger erfolgt nach Bedarf. Zur Inokulation werden die Blätter der Pflanzen (3-4 Wochen nach Überführung ins Gewächshaus) mit Sporensuspension des Pathogens tropfnass angesprüht. Anschließend werden die Pflanzen bei 100 % rel. Luftfeuchte und 10-20°C inkubiert. Nach 4-8 Tagen wird der Gesundheitszustand der Pflanzen anhand der befallenden Blattfläche in Prozent ermittelt.

Die transformierten Tabakpflanzen, in die ein erfindungsgemäßes CCoAMT-Gen eingesetzt wurde, zeigen einen deutlich geringeren Befall mit B. cinerea als die des nicht transformierten Pflanzen.

Erläuterungen der Abbildungen 1 bis 3 (Fig. 1 bis Fig. 3)

| Verwendete Abkürzungen: | |
|---|---|
| 1 | Anfang der codierenden Region |
| 2 | Ende der codierenden Region |
| CaMV | Cauliflower mosaic virus |
| Cb$^R$ | Carbenicillin-Resistenzgen |
| E | EcoRI-Schnittstelle |
| H | HindIII-Schnittstelle |
| Km$^R$ | Kanamycin-Resistenzgen für Pflanzen |
| P35S | CaMV35S-Promotor |
| pA35S | Polyadenylierungssequenz von CaMV |
| RV | EcoRV |
| S | SST1-Schnittstelle |
| Pfeilrichtung | Richtung des Promotors und des Gens |
| LB | linke Bordersequenz der T-DNA von A.-tumefaciens |
| RB | rechte Bordersequenz der T-DNA von A.-tumefaciens |

Fig. 1:    Fig. 1 stellt ein Schema des Plasmids pL2-4 dar, welches die proteincodierende Sequenz des CCoAMT-Gens (vergl. auch SEQ ID NO:1) auf dem EcoRI-Fragment enthält.

Fig. 2:    Fig. 2 stellt ein Schema des Plasmids pRT101:: CCoAMT dar, das ein chimäres CCoAMT-Gen enthält.

Fig. 3:    Fig. 3 stellt ein Schema des Plasmids pCV001:: CCoAMT dar, das ein chimäres CCoAMT-Gen enthält.

Bevorzuate Hybridisierungsbedingungen

Wie oben dargestellt sind die bevorzugten erfindungsgemäßen CCoAMT-Gene dadurch gekennzeichnet, daß sie mit der im Plasmid pL2-4 enthaltenen CCoAMT-cDNA-Sequenz oder ihren Teilen beziehungsweise mit der cDNA-Sequenz gemäß SEQ ID No: 1 oder ihren Teilen hybridisieren und für CCoAMT codieren.

Die Hybridisierung wird hierbei unter mäßig hohen stringenten Bedingungen, vorzugsweise bei 58 bis 65°C (besonders bevorzugt bei 63°C) in 3 bis 4 X konz. SSC in bekannter Weise durchgeführt.

Bei der Isolierung von CCoAMT-Genen aus anderen Quellen erhält man hierbei eine Population von cDNA's mit verwandten Sequenzen. Durch Expression z.B. in E. coli und Enzymaktivitätsmessungen (vergleiche z.B. Pakusch et.al., Arch. Biochem. Biosphys. (1989), 871 (S. 488-494) kann die gewünschte cDNA ausgewählt und nach bekannten Methoden isoliert werden.

SEQ ID NO:1

ART DER SEQUENZ: Nucleotid mit entsprechendem Protein

SEQUENZLÄNGE:1258 Basenpaare

STRANGFORM : Einzelstrang

TOPOLOGIE: linear

ART DES MOLEKÜLS: cDNA

URSPRÜNGLICHE HERKUNFT

ORGANISMUS: Petersilie

UNMITTELBARE EXPERIMENTELLE HERKUNFT

NAME DER ZELLINIE: Petersilie Zellkultur (Petroselinum crispum)

Merkmale: von 1    bis   370 BP 5' untranslatierte region
          von 371 bis  1093 BP reifes Peptid
          von1094 bis 1258 BP 3' untranslatierte Region

EIGENSCHAFTEN: cDNA für Caffeoyl-CoA-3-O-Methyltransferase aus Petersilie

```
  1  cgagctcagg cagatgcact taatcagcta accactgatg accttgaagg

 51  acagtttgca ttgctggaga cttcatcagt cgatgatgat cttgcgagtt

101  tgaagaaaga attgtctgga agtagaaaga aaggacagct tccgccagga

151  agaactactg ctgcctcaaa ctcgggtttt ccattcagag aaactgaaat

201  tgacaatgag ctaaacgaac ttaggagaaa agccgctgat tactaaatat

251  acaactctgc atatgttcac tatgactgca cctactgcat ctacaaatgt

301  actttttggt tgattgtgga cattctatac atacgttaag aggcagattt

351  gtcgtttgca caaattccag
```

```
371                   390                 410                 430
                        .                   .                   .
     atggcttctaatggtgaatctaaacattcagaagttgggcacaagagtctttttgcagagt
     MetAlaSerAsnGlyGluSerLysHisSerGluValGlyHisLysSerLeuLeuGlnSer

                   450                 470                 490
                     .                   .                   .
     gatgctctttatcagtatatacttgaaacaagtgtgtacccaagagaaccagaggcaatg
     AspAlaLeuTyrGlnTyrIleLeuGluThrSerValTyrProArgGluProGluAlaMet

                   510                 530                 550
                     .                   .                   .
     aaagagcttagagaagtcaccgcaaagcatccatggaatctgatgacaacatcagctgat
     LysGluLeuArgGluValThrAlaLysHisProTrpAsnLeuMetThrThrSerAlaAsp
```

```
              570                    590                    610
                 .                      .                      .
gaagggcagttcttgaacatgcttttgaagctcatcaatgccaaaaacaccatggagatt
GluGlyGlnPheLeuAsnMetLeuLeuLysLeuIleAsnAlaLysAsnThrMetGluIle

              630                    650                    670
                 .                      .                      .
ggtgtttacactggttattctctccttgccactgccctggctcttccagatgatggaaag
GlyValTyrThrGlyTyrSerLeuLeuAlaThrAlaLeuAlaLeuProAspAspGlyLys

              690                    710                    730
                 .                      .                      .
attttggcaatggatatcaacagagaaaactatgaaattggattacccatcattgaaaaa
IleLeuAlaMetAspIleAsnArgGluAsnTyrGluIleGlyLeuProIleIleGluLys

              750                    770                    790
                 .                      .                      .
gctggagttggtcacaaaattgacttcagagaaggcccagctttgcctgttcttgatcat
AlaGlyValGlyHisLysIleAspPheArgGluGlyProAlaLeuProValLeuAspHis

              810                    830                    850
                 .                      .                      .
atgcttgaagatggaaagtatcatggaacatttgattttgtatttgttgatgctgacaag
MetLeuGluAspGlyLysTyrHisGlyThrPheAspPheValPheValAspAlaAspLys

              870                    890                    910
                 .                      .                      .
gataactatatcaactaccacaagagattaattgatttagtaaaaatcggaggacttatc
AspAsnTyrIleAsnTyrHisLysArgLeuIleAspLeuValLysIleGlyGlyLeuIle

              930                    950                    970
                 .                      .                      .
ggctacgacaacaccctatggaatggttctgtggctcagccagctgatgctccaatgaga
GlyTyrAspAsnThrLeuTrpAsnGlySerValAlaGlnProAlaAspAlaProMetArg

              990                    1010                   1030
                 .                      .                      .
aagtatgtaaggtactacagagactttgtgattgagcttaacaaagctctggccgctgat
LysTyrValArgTyrTyrArgAspPheValIleGluLeuAsnLysAlaLeuAlaAlaAsp

              1050                   1070                   1090
                 .                      .                      .
cccaggattgagatctgtatgcttcctgttggtgatggacttaccctgtgccgtcgtatc
ProArgIleGluIleCysMetLeuProValGlyAspGlyValThrLeuCysArgArgIle


agc
Ser
```

```
1094         1110                   1130                   1150
                .                      .                      .
tgattatctaactgaaatttgagatattatttcacaatgtttttaacaaatggaatacttt

             1170                   1190                   1210
                .                      .                      .
tgctttgattgtatcttcctatgtttcttgttgaatttgcaatgtgcattattgatgatg

             1230                   1250     1258
                .                      .
aatatattcataattgatgttgaaaaaaaaaaaaaaaaaaaaaaa
```

**Patentansprüche**

1. Kaffeoyl-CoA3-O-Methyltransferase-Gene (CCoAMT-Gene).

2. CCoAMT-Gene, welche dadurch gekennzeichnet sind, daß sie mit der im Plasmid pL2-4 enthaltenen CCoAMT-cDNA Sequenz oder ihren Teilen bzw. mit der cDNA-Sequenz gemäß SEQ ID NO:1 oder ihren Teilen hybridisieren und für CCoAMT codieren.

3. CCoAMT-Gene gemäß den Ansprüchen 1 und 2, erhältlich aus dikotyledonen Pflanzen.

4. CCoAMT-Gene gemäß den Ansprüchen 1 bis 3, erhältlich aus Petersilie.

5. CCoAMT-Gene mit einer proteincodierenden Region, welche der cDNA entspricht, welche in dem Plasmid pL2-4 enthalten ist, bzw. im SEQ ID NO:1 aufgeführt ist, sowie die im wesentlichen gleichwirkenden DNA-Sequenzen.

6. CCoAMT-Gene gemäß den Ansprüchen 1 bis 5, welche als Promotor den TR-Promotor oder den 35S-Promotor enthalten.

7. CCoAMT-Gene gemäß den Ansprüchen 1 bis 6, mit einer proteincodierenden Region, welche der cDNA entspricht, die in dem Plasmid pL2-4 enthalten ist bzw. in SEQ ID NO:1 aufgeführt ist und mit dem TR-Promotor oder dem 35S-Promotor.

8. Regulatorisch wirkender Teil der CCoAMT-Gene gemäß den Ansprüchen 1 bis 5.

9. Struktur-Gene der CCoAMT-Gene sowie die DNA-Sequenzen, welche der cDNA entsprechen, die auf dem Plasmid pL2-4 enthalten ist bzw. in SEQ ID NO:1 aufgeführt wird, gemäß den Ansprüchen 1 bis 7.

10. Rekombinante prokaryontische oder eukaryontische DNA, welche ein oder mehrere CCoAMT-Gene oder ihre Teile gemäß den Ansprüchen 1 bis 9 als "fremde" DNA oder als "zusätzliche" DNA enthält.

11. Rekombinante DNA gemäß Anspruch 10, welche in Pflanzenzellen (einschließlich Protoplasten) oder Pflanzen (einschließlich Pflanzenteilen und Samen) enthalten ist.

12. Vektoren, welche ein oder mehrere CCoAMT-Gene oder ihre Teile gemäß den Ansprüchen 1 bis 9 und/oder rekombinante DNA gemäß Anspruch 10 enthalten.

13. Vektor-Plasmid pL2-4.

14. Transformierte Mikroorganismen, welche ein oder mehrere CCoAMT-Gene oder ihre Teile gemäß den Ansprüchen 1 bis 9 und/oder rekombinante DNA gemäß Anspruch 10 oder Vektoren gemäß den Ansprüchen 12 und 13 enthalten.

15. Escherichia coli Stamm E. coli DS pL2-4 sowie seine Mutanten.

16. Verwendung der CCoAMT-Gene und/oder ihrer Teile und/oder der rekombinanten DNA und/oder der Vektoren und/oder der transformierten Mikroorganismen gemäß den Ansprüchen 1 bis 15 zur Transformation von Pflanzenzellen (einschließlich Protoplasten) und Pflanzen (einschließlich Pflanzenteilen und Samen).

17. Transgene Pflanzenzellen (einschließlich Protoplasten) und Pflanzen (einschließlich Pflanzenteilen und Samen), welche ein oder mehrere CCoAMT-Gene und/oder deren Teile und/oder die rekombinante DNA gemäß den Ansprüchen 1 bis 10 als "fremde" oder "zusätzliche" DNA enthalten.

18. Verfahren zur Herstellung transgener Pflanzenzellen (einschließlich Protoplasten) und Pflanzen (einschließlich Pflanzenteile und Samen) mit erhöhter Resistenz gegen Schädlinge, dadurch gekennzeichnet, daß man

(a) ein oder mehrere CCoAMT-Gene oder ihre Teile und/oder die rekombinante DNA gemäß den Ansprüchen 1 bis 10 in den Genom von Pflanzenzellen (einschließlich Protoplasten) einsetzt und gegebenenfalls

(b) aus den transgenen Pflanzenzellen (einschließlich Protoplasten) vollständige transgene Pflanzen regeneriert und gegebenenfalls vermehrt und gegebenenfalls

(c) von den so erhaltenen transgenen Pflanzen der Elterngeneration oder weiterer daraus gewonnener Generationen die gewünschten Pflanzenteile (einschließlich Samen) gewinnt.

19. Verwendung der transgenen Pflanzenzellen (einschließlich Protoplasten) und Pflanzen (einschließlich Pflanzenteilen und Samen) gemäß Anspruch 17 zur Erzeugung von Vermehrungsmaterial sowie zur Erzeugung neuer Pflanzen, die die CCoAMT-Gene oder ihre Teile gemäß den Ansprüchen 1 bis 9 oder die rekombinante DNA gemäß Anspruch 10 enthalten und deren Vermehrungsmaterial.

20. Vermehrungsmaterial, erhältlich durch die Vermehrung der transgenen Pflanzenzellen und Pflanzen gemäß Anspruch 17.

21. Verwendung von DNA-Sequenzen, welche ganz oder teilweise der cDNA entsprechen, die auf dem Plasmid pL2-4 enthalten ist, bzw, in SEQ ID NO:1 aufgeführt ist, zur Isolierung von CCoAMT-Genen aus Pflanzen.

FIG.1

FIG. 2

FIG. 3